# EUROPEAN PATENT SPECIFICATION

(11) **EP 4 414 992 B1**
(45) Date of publication and mention of the grant of the patent: **04.11.2026**
(21) Application number: 22878445.0
(22) Date of filing: 30.09.2022
(51) Int. Cl.: G16C 20/30, G16C 20/70

(54) **LOW-MOLECULAR-WEIGHT-COMPOUND SEARCH METHOD, PROGRAM, DEVICE, AND SYSTEM**
SUCHVERFAHREN FÜR VERBINDUNG MIT NIEDRIGEM MOLEKULARGEWICHT, PROGRAMM, VORRICHTUNG UND SYSTEM
PROCÉDÉ, PROGRAMME, DISPOSITIF ET SYSTÈME DE RECHERCHE DE COMPOSÉS DE FAIBLE POIDS MOLÉCULAIRE

(30) Priority: 04.10.2021 JP 2021163292
(43) Date of publication of application: 14.08.2024
(73) Proprietor: DAIKIN INDUSTRIES, LTD., Osaka-shi, Osaka 530-0001 (JP)
(72) Inventor: TACHIKAWA, Shohei, Osaka-shi, Osaka 530-8323 (JP)
(74) Representative: Hoffmann Eitle
(86) International application number: PCT/JP2022/036775
(87) International publication number: WO 2023/058576

(56) References cited:
- WO-A1-2019/172280
- WO-A1-2020/176164
- WO-A1-2021/079985
- JP-A- 2021 174 473
- JP-A- 2021 196 710
- US-A1- 2021 271 980
- DAVID E GRAFF ET AL: "Accelerating high-throughput virtual screening through molecular pool-based active learning", ARXIV.ORG, CORNELL UNIVERSITY LIBRARY, 201 OLIN LIBRARY CORNELL UNIVERSITY ITHACA, NY 14853, 13 December 2020 (2020-12-13), XP081917274, DOI: 10.1039/D0SC06805E
- LEE SANGWON ET AL: "Machine learning-based discovery of molecules, crystals, and composites: A perspective review", KOREAN JOURNAL OF CHEMICAL ENGINEERING, SPRINGER US, NEW YORK, vol. 38, no. 10, 9 September 2021 (2021-09-09), pages 1971 - 1982, XP037579854, ISSN: 0256-1115, [retrieved on 20210909], DOI: 10.1007/S11814-021-0869-2
- HIE BRIAN ET AL: "Leveraging Uncertainty in Machine Learning Accelerates Biological Discovery and Design", CELL SYSTEMS, vol. 11, no. 5, 15 October 2020 (2020-10-15), US, pages 461 - 477.e9, XP055950712, ISSN: 2405-4712, Retrieved from the Internet <URL:http://dx.doi.org/10.1016/j.cels.2020.09.007> DOI: 10.1016/j.cels.2020.09.007
- JABLONKA KEVIN MAIK ET AL: "Big-Data Science in Porous Materials: Materials Genomics and Machine Learning", CHEMICAL REVIEWS, vol. 120, no. 16, 10 June 2020 (2020-06-10), pages 8066 - 8129, XP093337592, ISSN: 0009-2665, Retrieved from the Internet <URL:http://pubs.acs.org/doi/pdf/10.1021/acs.chemrev.0c00004> DOI: 10.1021/acs.chemrev.0c00004

## Description

### Technical Field

The present disclosure relates to a method, a program, an apparatus, and a system for searching a low molecular weight compound.

### Background Art

Conventionally, in order to search for a material that achieves a desired function, characteristic, or physical property, an experimenter has had to conduct an experiment based on the knowledge and experience of the experimenter. However, in such a material searching method, the success of the material searching is affected by the knowledge and experience of the experimenter.

For this reason, in recent years, a technique referred to as materials informatics, which uses information science to search for materials that achieve the desired function, characteristic, and physical property, is now known (Patent Document 1).

### Citation List

### Patent Document

Patent document 1: Japanese Unexamined Patent Application Publication No. 2020-71827
DAVID E GRAFF ET AL: "Accelerating high-throughput virtual screening through molecular pool-based active learning", ARXIV.ORG, CORNELL UNIVERSITY LIBRARY, 201 OLIN LIBRARY CORNELL UNIVERSITY ITHACA, NY 14853, 13 December 2020 (2020-12-13) discloses an assessment of various surrogate model architectures, acquisition functions, and acquisition batch sizes as applied to several protein-ligand docking datasets.
LEE SANGWON ET AL: "Machine learning-based discovery of molecules, crystals, and composites: A perspective review", KOREAN JOURNAL OF CHEMICAL ENGINEERING, SPRINGER US, NEW YORK, vol 38, no. 10, 9 September 2021 (2021-09-09) discloses various models used to represent molecules and crystals that are introduced and such representations can be used within the neural networks to generate materials that satisfy specified physical features and properties.
US 2021/271980 A1 discloses a computer-implemented method of generating objects with a deterministic decoder variational autoencoder.
Hie Brian ET AL: "Leveraging Uncertainty in Machine Learning Accelerates Biological Discovery and Design", Cell Systems, vol. 11, no. 5, 15 October 2020 (2020-10-15), pages 461-477.e9, XP055950712 discloses a method of robust uncertainty prediction in biological discovery by leveraging Gaussian process-based uncertainty prediction on modern pre-trained features, whereby a model is trained on just 72 compounds to make predictions over a 10,833-compound library, identifying and experimentally validating compounds with nanomolar affinity for diverse kinases and whole-cell growth inhibition of Mycobacterium tuberculosis.
Jablonka Kevin Maik ET AL: "Big-Data Science in Porous Materials: Materials Genomics and Machine Learning", Chemical Reviews, vol. 120, no. 16, 10 June 2020 (2020-06-10), pages 8066-8129, XP093337592 is a review which discloses big-data methods as a powerful technique to study materials and to discover complex correlations, whereby the first part of the review gives an introduction to the principles of big-data science and in the second part it is reviewed how the different approaches of machine learning have been applied to porous materials.

### Summary of Invention

### Technical Problem

However, in Patent Document 1, the target of the search is a polymer (a polymer compound), and is not a low molecular weight compound. In the present disclosure, an object is to search for a low molecular weight compound having a high possibility of having a desired physical property.

### Solution to Problem

The method according to the first aspect of the present disclosure is realized by the independent claim, the dependent claims concern particular embodiments.

According to the first aspect of the present disclosure, it is possible to search for a low molecular weight compound that is highly likely to have the desired physical property.

### Brief Description of Drawings

[FIG. 1] FIG. 1 is a diagram illustrating an overall configuration according to an embodiment of the present disclosure.
[FIG. 2] FIG. 2 is a diagram illustrating a hardware configuration of a compound search apparatus according to an embodiment of the present disclosure.
[FIG. 3] FIG. 3 is a diagram for explaining the overall flow according to an embodiment of the present disclosure.
[FIG. 4] FIG. 4 is a diagram for explaining the overall flow according to an embodiment of the present disclosure.
[FIG. 5] FIG. 5 is a diagram for explaining the substitution of chemical structures with a plurality of types of descriptors according to an embodiment of the present disclosure.
[FIG. 6] FIG. 6 is a diagram for explaining the selection of the most diverse group of compounds according to an embodiment of the present disclosure.

### Description of Embodiments

Embodiments of the present disclosure will be described below based on the drawings.

### < Terminology >

As used herein, a "low molecular weight compound" is a compound having a molecular weight of 10,000 or less.

### < Overall configuration >

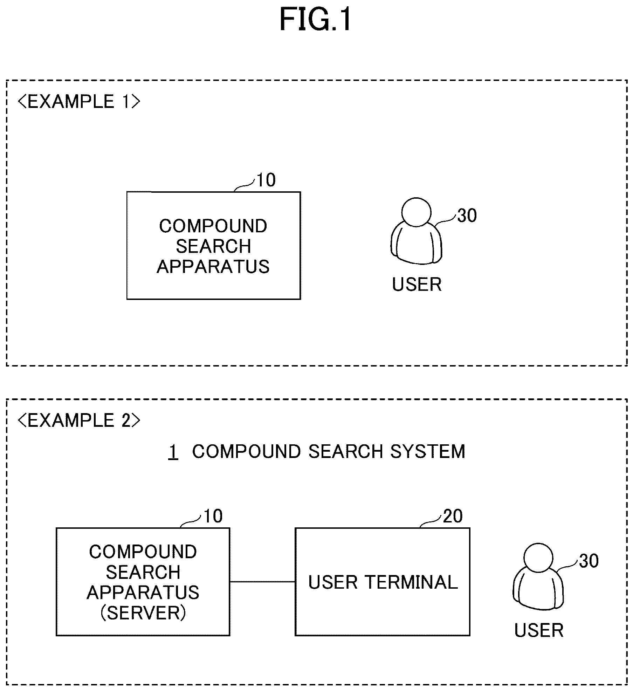

FIG. 1 is a diagram illustrating an overall configuration according to an embodiment of the present disclosure. Hereinafter, the description will be divided into Example 1 and Example 2.

### < Example 1 >

As illustrated in the upper part of FIG. 1, in Example 1, a user 30 can operate a compound search apparatus 10 to have the compound search apparatus 10 search for a compound.

The compound search apparatus 10 performs a process of searching for a compound. Specifically, the compound search apparatus 10 receives an instruction input by the user 30, performs a process for searching for a compound, and presents the result of the search process. The compound search apparatus 10 is, for example, a personal computer, a tablet, a smartphone, etc.

### < Example 2 >

As illustrated in the lower part of FIG. 1, in Example 2, the user 30 can operate a user terminal 20 to have the compound search apparatus 10 (for example, a server) search for a compound. A compound search system 1 includes the compound search apparatus 10 (for example, a server) and the user terminal 20.

The compound search apparatus 10 (for example, a server) performs a process of searching for a compound. Specifically, the compound search apparatus 10 (for example, a server) performs a process of searching for a compound in response to a request from the user terminal 20, and causes the user terminal 20 to present the result of the search process. The compound search apparatus 10 (for example, a server) includes one or more computers. The compound search apparatus 10 (for example, a server) can transmit and receive data to and from the user terminal 20 via any network.

The user terminal 20 requests the compound search apparatus 10 (for example, a server) to perform a process of searching for a compound and presents the result of the search. Specifically, the user terminal 20 transmits an instruction input by the user 30 to the compound search apparatus 10 (for example, a server). The user terminal 20 receives and presents the result of the process of searching for the compound from the compound search apparatus 10 (for example, a server). The user terminal 20 is, for example, a personal computer, a tablet, a smartphone, etc. The user terminal 20 can transmit and receive data to and from the compound search apparatus 10 (for example, a server) via any network.

### < Hardware configuration >

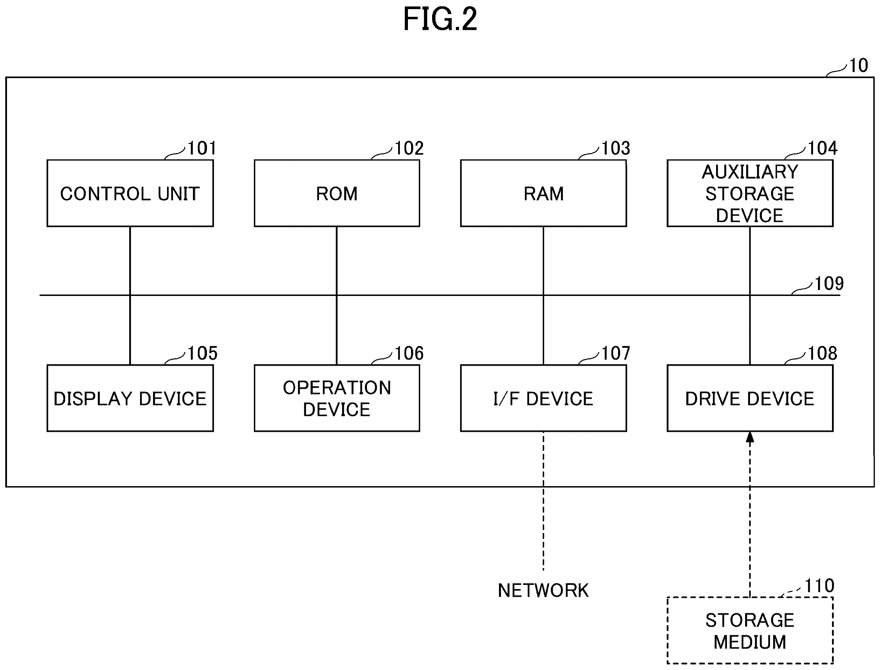

FIG. 2 is a diagram illustrating a hardware configuration of the compound search apparatus 10 according to an embodiment of the present disclosure. The same applies to the user terminal 20.

The compound search apparatus 10 includes a control unit 101, a ROM (Read Only Memory) 102, and a RAM (Random Access Memory) 103. The control unit 101, the ROM 102, and the RAM 103 form what is referred to as a computer. The compound search apparatus 10 may also include an auxiliary storage device 104, a display device 105, an operation device 106, an I/F (interface) device 107, and a drive device 108. The pieces of hardware of the compound search apparatus 10 are connected to each other via a bus 109.

The control unit (for example, a CPU) 101 is an arithmetic device for executing various programs installed in the auxiliary storage device 104. When the control unit 101 executes the programs, the processes described herein are executed.

The ROM 102 is a nonvolatile memory. The ROM 102 functions as a main storage device for storing various programs, data, etc., necessary for the control unit 101 to execute various programs installed in the auxiliary storage device 104. Specifically, the ROM 102 functions as a main storage device for storing boot programs such as BIOS (Basic Input/Output System) and EFI (Extensible Firmware Interface).

The RAM 103 is a volatile memory such as DRAM (Dynamic Random Access Memory) or SRAM (Static Random Access Memory). The RAM 103 functions as a main storage device that provides a work area that is expanded when various programs installed in the auxiliary storage device 104 are executed by the control unit 101.

The auxiliary storage device 104 is an auxiliary storage device that stores various programs and information used when various programs are executed.

The display device 105 is a display device that displays the internal state or the like of the compound search apparatus 10.

The operation device 106 is an input device to which an operator of the compound search apparatus 10 inputs various instructions to the compound search apparatus 10.

The I/F device 107 is a communication device for connecting to a network and communicating with other devices.

The drive device 108 is a device for setting a storage medium 110. The storage medium 110 includes a medium for recording information optically, electrically, or magnetically, such as a CD-ROM, a flexible disk, a magneto-optical disk or the like. The storage medium 110 may include a semiconductor memory or the like for recording information electrically, such as a ROM, a flash memory or the like.

The various programs installed in the auxiliary storage device 104 are installed, for example, when the distributed storage medium 110 is set in the drive device 108 and the various programs recorded in the storage medium 110 are read out by the drive device 108. Alternatively, the various programs installed in the auxiliary storage device 104 may be downloaded from the network via the I/F device 107 to be installed.

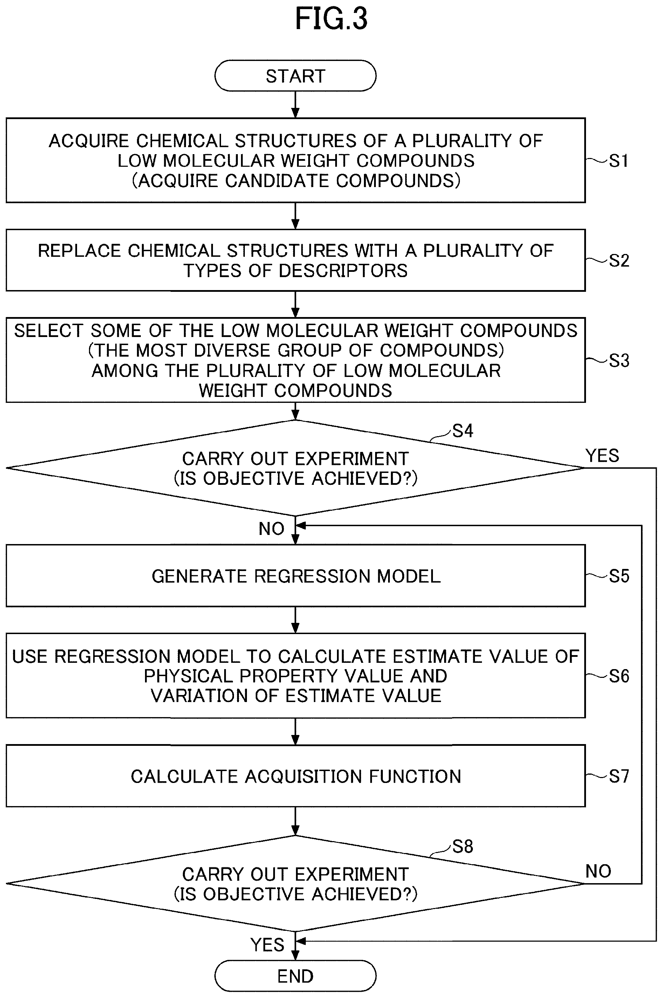

The process executed by the control unit 101 will be described below. The entire flow will be described with reference to FIG. 3, and then the details of each processing will be described.

### < Overview of the entire process >

FIG. 3 is a diagram for explaining the entire process according to an embodiment of the present disclosure. Note that the compound search apparatus 10 acquires a target value (for example, a target value entered by the experimenter, etc.) of the physical property value of the low molecular weight compound.

In step 1 (S1), the compound search apparatus 10 acquires the chemical structures of a plurality of low molecular weight compounds. That is, the compound search apparatus 10 acquires candidate compounds which are compounds to be candidates.

In step 2 (S2), the compound search apparatus 10 replaces the chemical structures of the plurality of low molecular weight compounds acquired in S1 with a plurality of types of descriptors (for example, the RDKit2D descriptor).

In step 3 (S3), the compound search apparatus 10 selects some of the low molecular weight compounds (for example, the most diverse group of compounds) among the plurality of low molecular weight compounds acquired in S1, based on the plurality of types of descriptors obtained in S2. Specifically, the compound search apparatus 10 selects the most diverse group of compounds by using the D-optimization technique.

In step 4 (S4), the experimenter carries out an experiment by using the most diverse group of compounds selected in S3. As a result of the experiment, if the objective is achieved (if the measured value of the physical property value satisfies the target value of the physical property value), the process is ended, and if the objective is not achieved, the process proceeds to step 5.

In step 5 (S5), the compound search apparatus 10 generates a regression model (specifically, a Gaussian process regression model) by using the most diverse group of compounds selected in S3. Specifically, the compound search apparatus 10 generates a regression model by using the chemical structures of the low molecular weight compounds selected in S3 and the measured values of the physical property value of the low molecular weight compounds selected in S3 (values obtained by the experiment in S4). The regression model is a model (that is, a model for estimating the "physical property value" from the "chemical structure of low molecular weight compounds") in which the input is the "chemical structure of the low molecular weight compound" and the output is the "physical property value".

Hereinafter, in steps 6 and 7, the compound search apparatus 10 calculates an acquisition function by using a Bayesian optimization method.

In step 6 (S6), the compound search apparatus 10 uses the regression model (specifically, a Gaussian process regression model) generated in S5 to calculate an estimate value of a physical property value and a variation of the estimate value from the chemical structure of the low molecular weight compounds other than those in the most diverse group of compounds selected in S3 among the plurality of low molecular weight compounds obtained in S1.

In step 7 (S7), the compound search apparatus 10 calculates an acquisition function from the estimate value of the physical property value calculated in S6 and the variation of the estimate value. Thereafter, the compound search apparatus 10 determines a low molecular weight compound for which the acquisition function is the maximum or minimum (for example, if the acquisition function is expected improvement, the low molecular weight compound having the maximum expected improvement). The chemical structure of the low molecular weight compound having the maximum or minimum acquisition function is displayed on the compound search apparatus 10 or the user terminal 20.

In step 8 (S8), the experimenter carries out an experiment by using the low molecular weight compound determined based on the acquisition function calculated in S7 (for example, if the acquisition function is expected improvement, the low molecular weight compound having the maximum expected improvement). As a result of the experiment, when the objective is achieved (when the measured value of the physical property value meets the target value of the physical property value), the process is ended, and when the objective is not achieved, the process returns to step 5, and the regression model is generated again by adding the value obtained by the experiment in S8, (that is, the compound search apparatus 10 adds the chemical structures of the low molecular weight compounds subjected to the experiment in S8 and the measured values of the physical property value of the low molecular weight compounds subjected to the experiment in S8 (the values obtained by the experiment in S8) to the data set for machine learning and relearns the regression model).

The details of each process will be described below.

### << Acquisition of a plurality of low molecular weight compounds (S1 in FIG. 3) >>

First, the acquisition of a plurality of low molecular weight compounds will be described. The compound search apparatus 10 acquires the chemical structures of a plurality of low molecular weight compounds (candidate compounds that are compounds to be candidates). The following description will be divided into [compounds selected by a person], [compounds extracted from a database], and [compounds designed by a specific algorithm].

### [Compounds selected by a person]

For example, the compound search apparatus 10 acquires a plurality of low molecular weight compounds selected by a person (for example, the experimenter) as candidate compounds. In this case, compounds utilizing the knowledge of a person (for example, the experimenter) can be used as candidate compounds.

### [Compounds extracted from the database]

For example, the compound search apparatus 10 acquires a plurality of low molecular weight compounds extracted from any compound database as candidate compounds. In this case, various compounds in the compound database can be used as candidate compounds.

### [Compounds designed by a specific algorithm]

For example, the compound search apparatus 10 acquires a plurality of low molecular weight compounds designed by a specific algorithm as candidate compounds. For example, the algorithm may be an algorithm that fragments molecules into substructures, an algorithm that fragments molecules into substructures and then connects those substructures, or an algorithm that lists arbitrary substructures and then connects those substructures.

### << Replacement of chemical structure with descriptor (S2 in FIG. 3) >>

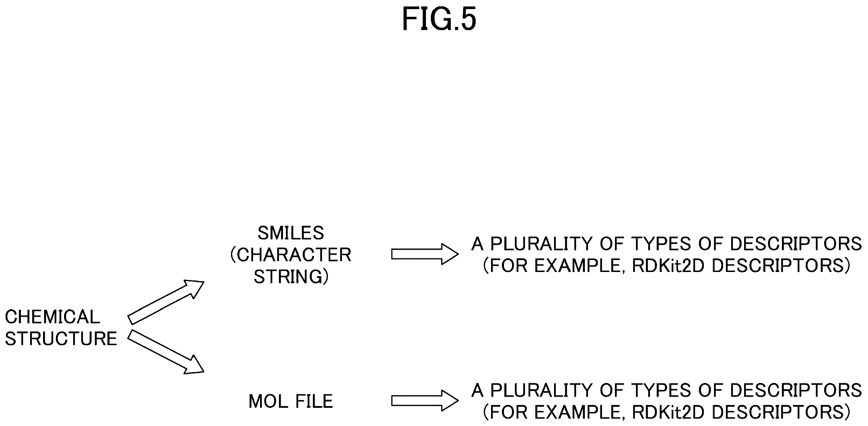

Next, the replacement of chemical structures with descriptors will be described. The compound search apparatus 10 replaces chemical structures of low molecular weight compounds with a plurality of types of descriptors. The descriptors of chemical structures of compounds are those that quantify the characteristics of compounds. The descriptors will be described in detail with reference to FIG. 5.

FIG. 5 is a diagram for explaining the substitution of a chemical structure with a plurality of types of descriptors according to an embodiment of the present disclosure.

For example, the compound search apparatus 10 can convert a chemical structure of a low molecular weight compound into a character string (for example, a character string of SMILES (simplified molecular input line entry system)) and replace the character string with a plurality of types of descriptors. The description method of the chemical structure is not limited to SMILES and may be another description method (for example, a MOL file).

For example, the compound search apparatus 10 can replace the chemical structure of a plurality of low molecular weight compounds with a plurality of types of RDKit2D descriptors.

### << Selection of the most diverse group of compounds (S3 in FIG. 3) >>

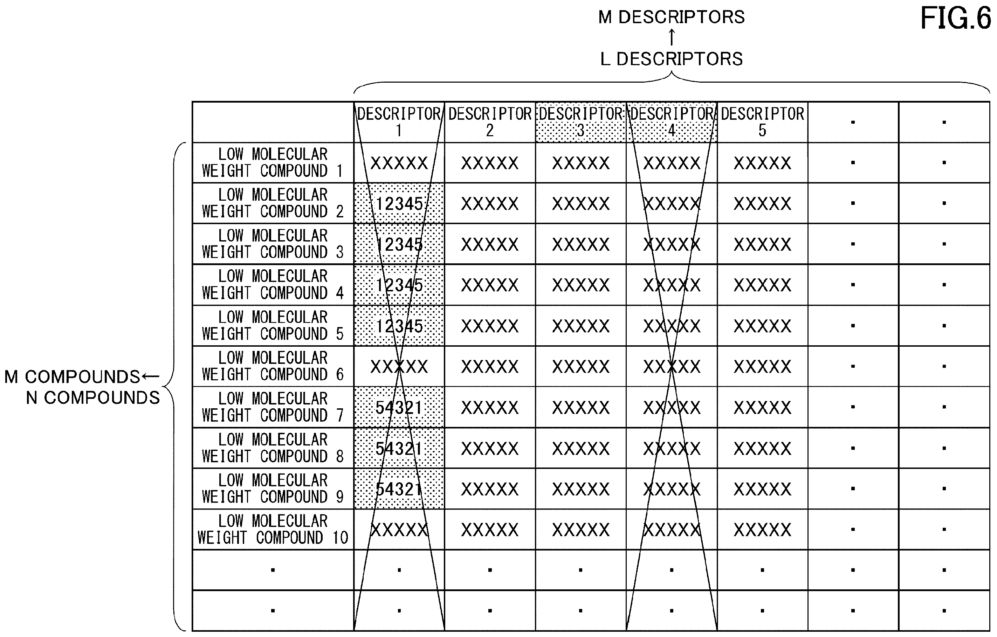

Next, the selection of the most diverse group of compounds will be described. The data set for machine learning is preferably a data set of a diverse group of compounds. If the compounds in the dataset for machine learning were all similar compounds (that is, the descriptors are similar), then the application range of the model generated by machine learning would be small (that is, if a model is generated by using only a certain compound, no other compounds can be estimated). Therefore, the compound search apparatus 10 selects the most diverse group of compounds among a plurality of low molecular weight compounds based on a plurality of types of descriptors by using a D-optimization technique. The description will be described in detail with reference to FIG. 6.

FIG. 6 is a diagram for explaining the selection of the most diverse group of compounds according to an embodiment of the present disclosure. It is assumed that each of a plurality of low molecular weight compounds (low molecular weight compound 1, low molecular weight compound 2, low molecular weight compound 3, low molecular weight compound 4, low molecular weight compound 5, low molecular weight compound 6, low molecular weight compound 7, low molecular weight compound 8, low molecular weight compound 9, low molecular weight compound 10, ..., low molecular weight compound N in FIG. 6) is replaced by a plurality of types of descriptors (descriptor 1, descriptor 2, descriptor 3, descriptor 4, descriptor 5, ..., descriptor L of FIG. 6). It is assumed that M low molecular weight compounds (the most diverse group of M compounds) are selected from N low molecular weight compounds (N candidate compounds). It is also assumed that the number of a plurality of types of descriptors is L.

The compound search apparatus 10 can select the most diverse group of compounds from among the plurality of low molecular weight compounds by selecting a combination in which det (X^{T}X) is maximum for the plurality of types of descriptors. The det (X^{T}X) is the D optimization criterion (determinant: det (X^{T}X) is the value calculated from X^{T}X (formula (1) below is a matrix of X^{T}X)).
[Formula 1] $\begin{matrix}DESCRIPTOR \\ DESCRIPTOR \\ \\ DESCRIPTOR\end{matrix} \left(\begin{matrix}{x}_{1}^{\left(1\right)} & {x}_{1}^{\left(2\right)} & … & {x}_{1}^{\left(M\right)} \\ {x}_{2}^{\left(1\right)} & {x}_{2}^{\left(2\right)} & … & {x}_{2}^{\left(M\right)} \\ … & … & … & … \\ {x}_{M}^{\left(1\right)} & {x}_{M}^{\left(2\right)} & … & {x}_{M}^{\left(M\right)}\end{matrix}\right) \begin{matrix}DESCRIPTOR DESCRIPTOR DESCRIPTOR \\ \left(\begin{matrix}{x}_{1}^{\left(1\right)} & {x}_{2}^{\left(1\right)} & ⋮ & {x}_{M}^{\left(1\right)} \\ {x}_{1}^{\left(2\right)} & {x}_{2}^{\left(2\right)} & ⋮ & {x}_{M}^{\left(2\right)} \\ ⋮ & ⋮ & ⋮ & ⋮ \\ {x}_{1}^{\left(M\right)} & {x}_{2}^{\left(M\right)} & ⋮ & {x}_{M}^{\left(M\right)}\end{matrix}\right)\end{matrix}$ FORMULA (1)

Here, in order to prevent det (X^{T}X) = 0, it is essential that the number of the most diverse compound groups (M) ≥ the number of descriptors. Therefore, the compound search apparatus 10 narrows down the number of descriptors to M. Then, a matrix of N×M is formed. The compound search apparatus 10 selects the combination with the maximum value of det (X^{T}X) among _{N}C_{M} versions of det (X^{T}X). For example, the compound search apparatus 10 may use a genetic algorithm to select the combination with the maximum value of det (X^{T}X) among _{N}C_{M} versions of det (X^{T}X).

### << Narrowing down to descriptors with a large amount of information>>

In narrowing down the descriptors, it is preferable that a descriptor having a large amount of information remains. Therefore, the compound search apparatus 10 narrows down the descriptors by using the following method. Hereinafter, the description will be divided into [deletion of descriptors having a high ratio of same values] and [selecting some of highly correlated descriptors].

### [Deletion of descriptors having a high ratio of same values]

The compound search apparatus 10 deletes descriptors having a high ratio of the same value (for example, descriptor 1 of FIG. 6). Specifically, the compound search apparatus 10 deletes descriptors for which the ratio of the low molecular weight compounds having the same value to all low molecular weight compounds is larger than a threshold value.

### [Selecting some of highly correlated descriptors]

The compound search apparatus 10 selects some of descriptors that are highly correlated, with respect to a plurality of types of descriptors (for example, one descriptor (descriptor 4) in a set of highly correlated descriptors (descriptor 3 and descriptor 4 in FIG. 6) is deleted). Specifically, with respect to each of the descriptors in a set of highly correlated descriptors, the compound search apparatus 10 calculates the correlation coefficients with all other descriptors, converts the correlation coefficients into absolute values, and deletes the descriptor whose value of the sum of all absolute values is larger.

The descriptors will now be described. For example, descriptors may include at least one of the molecular weight, the molecular weight calculated for atoms other than hydrogen atoms, the number of valence electrons, the number of atoms other than hydrogen atoms, the sum of nitrogen atoms and "hydrogen bonded to nitrogen atoms" and hydroxyl groups, the sum of nitrogen atoms and oxygen atoms, the number of hydrogen atoms and "atoms other than carbon atoms", the number of rotatable bonds, the number of rings, the number of radicals, the octanol/water partition coefficient, the number of spiro atoms, the number of bridgehead atoms, the number of aliphatic carbon rings, the number of aliphatic heterocycles, the number of aliphatic rings, the number of aromatic carbons, the number of aromatic heterocycles, the number of aromatic rings, the number of hydrogen acceptor groups, the number of hydrogen donor groups, the number of saturated carbon rings, the number of saturated heterocycles, the number of saturated rings, the number of rings, the number of aliphatic carboxylic acids, the number of aliphatic hydroxyl groups, the number of nitrogen functional groups bonded to aromatic compounds, the number of aromatic carboxylic acids, the number of aromatic nitrogens, the number of aromatic amines, the number of aromatic hydroxyl groups, the number of carboxylic acids, the number of ester groups, the number of carbonyl groups, the number of carbonyl groups excluding carboxylic acids, the number of thiocarbonyl groups, the number of imines, the number of tertiary amines, the number of secondary amines, the number of primary amines, the number of hydroxylamino groups, the number of pyrrole structures, the number of thiol groups, the number of aldehydes, the number of alkyl carbamates, the number of alkyl halides, the number of amides, the number of amidines, the number of anilines, the number of azide groups, the number of barbituric acid structures, the number of benzene rings, the number of bicyclic structures, the number of diazo groups, the number of dihydropyridines, the number of epoxide rings, the number of esters, the number of ether oxygens, the number of furan rings, the number of guanidine groups, the number of halogens, the number of hydrazine groups, the number of imidazole rings, the number of imide groups, the number of isocyanates, the number of isothiocyanates, the number of ketones, the number of beta lactams, the number of cyclic esters, the number of methoxy groups, the number of morpholine rings, the number of nitriles, the number of nitro groups, the number of nitrobenzene structures, the number of non-orthonitrobenzene structures, the number of oxazole rings, the number of phenol structures, the number of phosphate groups, the number of phosphate ester groups, the number of piperidine rings, the number of piperidine rings, the number of primary amides, the number of pyridine rings, the number of quaternary nitrogens, the number of thioethers, the number of sulfonamides, the number of sulfone groups, the number of terminal acetylenes, the number of tetrazole rings, the number of thiazole rings, the number of thiophene rings, or the number of urea structures.

### << Estimation of physical property value and calculation of variation (S6 in FIG. 3) >>

Next, the estimate value of the physical property value and the calculation of the variation of the estimate value will be described.

The compound search apparatus 10 calculates the estimate value of the physical property value of each low molecular weight compound and the variation of the estimate value from the chemical structure of each low molecular weight compound by using a regression model (specifically, a Gaussian process regression model). That is, the compound search apparatus 10 outputs a probability distribution of the physical property value (the estimate value is the average value of the probability distribution, and the variation of the estimate value is the standard deviation of the probability distribution).

### << Calculation of the acquisition function (S7 in FIG. 3) >>

Next, calculation of the acquisition function will be described. The compound search apparatus 10 calculates the acquisition function from the estimate value of the physical property value and the variation of the estimate value. The compound search apparatus 10 can determine the low molecular weight compound for which the acquisition function is the maximum or minimum.

For example, the acquisition function is expected improvement (EI). When the acquisition function is expected improvement (EI), the compound search apparatus 10 can select a compound that has a high possibility of satisfying a desired physical property by determining a low molecular weight compound that has the maximum expected improvement (EI). Note that the acquisition function is not limited to expected improvement (EI). The types of acquisition functions will be described below.

### [Acquisition function]

The acquisition function may be any of the following four types. EI is the expected improvement, PI is the improvement probability, UCB is the upper confidence limit, and LCB is the lower confidence limit. When the acquisition function is EI, PI, and UCB, the low molecular weight compound with the highest EI, PI, and UCB is determined. When the acquisition function is LCB, the low molecular weight compound with the lowest LCB is determined. EI, PI, UCB, and LCB are calculated by using the following formulas.

1. EI (Expected Improvement) $EI \left(x\right) = \left(μ\left(x\right)-{y}_{max}\right) Φ \left(Z\right) + σ \left(x\right) φ \left(Z\right) if σ \left(x\right) > 0$ $EI \left(x\right) = 0 if σ \left(x\right) = 0$ Note that Φ is the cumulative density function (the value obtained by integrating the probability density function from -∞ to a specific value), φ is the probability density function, and Z indicates (yₘₐₓ - µ)/σ(x). The notation of µ is the average value, and σ is the standard deviation.
2. PI (Probability of Improvement) $PI \left(x\right) = {{\int }^{∞}}_{ymax} N \left(f\left|μ\left({x}^{2}\right), σ\left(x\right)\right.\right) df$ Note that f~N (f|µ, σ²) is the estimated result by a Gaussian process, and f~N (f|µ, σ²) indicates that the probability variable f follows a normal distribution with average value µ and variation σ². The notation of µ is the average value and σ is the standard deviation.
3. UCB (Upper Confidence Bound) $UCB \left(x\right) = μ \left(x\right) + kσ \left(x\right)$ Note that µ(x) is the term corresponding to the exploitation and σ(x) is the term corresponding to the search. The notation of k indicates the degree of the importance placed on the search. The notation of µ is the average value and σ is the standard deviation.
4. LCB (Lower Confidence Bound) $LCB \left(x\right) = μ \left(x\right) - kσ \left(x\right)$ Note that µ (x) is the term corresponding to the utilization, and σ(x) is the term corresponding to the search. The notation of k indicates the degree of the importance placed on the search. The notation of µ is the average value and σ is the standard deviation.

Thus, by using a Bayesian optimization method, the compound search apparatus 10 can select compounds that are highly likely to satisfy the desired physical property.

### < Other Embodiment >

The Bayesian optimization applied to simultaneous experiments of multiple compounds will be described below.

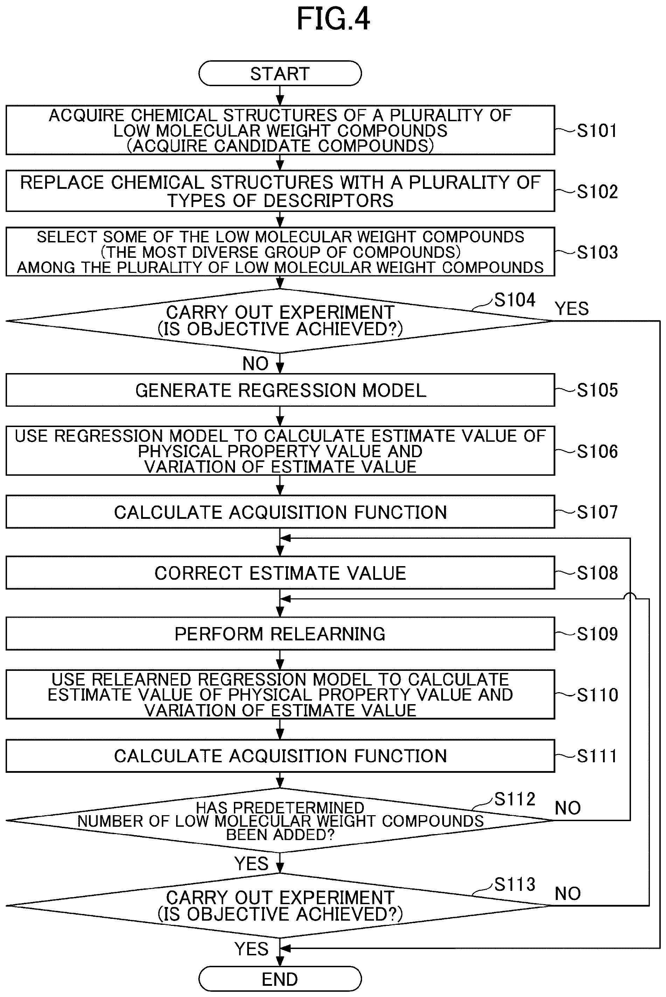

FIG. 4 is a diagram for explaining the overall flow according to an embodiment of the present disclosure. Note that the compound search apparatus 10 acquires a target value (for example, the target value input by the experimenter, etc.) of the physical property value of the low molecular weight compound.

In step 101 (S101), the compound search apparatus 10 acquires the chemical structures of a plurality of low molecular weight compounds. That is, the compound search apparatus 10 acquires candidate compounds that are compounds to be candidates.

In step 102 (S102), the compound search apparatus 10 replaces the chemical structures of the plurality of low molecular weight compounds acquired in S101 with a plurality of types of descriptors (for example, the RDKit2D descriptor).

In step 103 (S103), the compound search apparatus 10 selects some of the low molecular weight compounds (for example, the most diverse group of compounds) among the plurality of low molecular weight compounds acquired in S101 based on the plurality of types of descriptors obtained in S102. Specifically, the compound search apparatus 10 selects the most diverse group of compounds by using a D-optimization technique.

In step 104 (S104), the experimenter performs an experiment by using the most diverse group of compounds selected in S103. As a result of the experiment, when the objective is achieved (if the measured value of the physical property value satisfies the target value of the physical property value), the process is ended, and when the objective is not achieved, the process proceeds to step 105.

In step 105 (S105), the compound search apparatus 10 generates a regression model (specifically, a Gaussian process regression model) by using the most diverse group of compounds selected in S103. Specifically, the compound search apparatus 10 generates a regression model by using the chemical structures of the low molecular weight compounds selected in S103 and the measured values of the physical property value of the low molecular weight compounds selected in S103 (values obtained by the experiment in S104). The regression model is a model (that is, a model for estimating the "physical property value" from the "chemical structure of low molecular weight compounds") in which the input is the "chemical structure of the low molecular weight compound" and the output is the "physical property value".

Hereinafter, in steps 106 and 107, the compound search apparatus 10 calculates an acquisition function by using a Bayesian optimization method.

In step 106 (S106), the compound search apparatus 10 calculates the estimate value of the physical property value and the variation of the estimate value from the chemical structure of the low molecular weight compound other than the most diverse group of compounds selected in S103 among the plurality of low molecular weight compounds obtained in S101, by using the regression model (specifically, a Gaussian process regression model) generated in S105.

In step 107 (S107), the compound search apparatus 10 calculates the acquisition function from the estimate value of the physical property value calculated in S106 and the variation of the estimate value. Thereafter, the compound search apparatus 10 determines the low molecular weight compound for which the acquisition function is the maximum or minimum (for example, if the acquisition function is expected improvement, the low molecular weight compound having the maximum expected improvement). The chemical structure of the low molecular weight compound for which the acquisition function is the maximum or minimum is displayed on the compound search apparatus 10 or the user terminal 20.

In step 108 (S108), the compound search apparatus 10 corrects the estimate value (specifically, increases or decreases the estimate value) of the physical property value calculated in S106 of the low molecular weight compound determined in S107. For example, the compound search apparatus 10 corrects the estimate value of the physical property value according to an instruction for correction input by a person (for example, an experimenter). For example, the compound search apparatus 10 corrects the estimate value of the physical property value based on a predetermined rule.

In step 109 (S109), the compound search apparatus 10 adds the estimate value corrected in S108 and generates the regression model again (that is, the compound search apparatus 10 adds the chemical structure of the low molecular weight compound whose estimate value has been corrected in S108 and the estimate value corrected in S108 (estimate value after correction) to the data set for machine learning and relearns the regression model).

In step 110 (S110), the compound search apparatus 10 calculates the estimate value of the physical property value and the variation of the estimate value from the chemical structure of the low molecular weight compounds other than the most diverse group of compounds selected in S103 among the plurality of low molecular weight compounds obtained in S101, by using the regression model (specifically, a Gaussian process regression model) relearned in S109.

In step 111 (S111), the compound search apparatus 10 calculates the acquisition function from the estimate value of the physical property value and the variation of the estimate value calculated in S110. Thereafter, the compound search apparatus 10 determines the low molecular weight compound for which the acquisition function is the maximum or minimum (for example, if the acquisition function is expected improvement, the low molecular weight compound having the maximum expected improvement). The chemical structure of the low molecular weight compound for which the acquisition function is the maximum or minimum is displayed on the compound search apparatus 10 or the user terminal 20.

In the present embodiment, the experimenter carries out an experiment by using the low molecular weight compound determined in S107 and the low molecular weight compound determined in S111. That is, by repeating steps S108 to S112, the low molecular weight compounds to be subjected to the experiment are added.

In step 112 (S112), the compound search apparatus 10 determines whether a predetermined number of low molecular weight compounds (that is, the number of low molecular weight compounds to be subjected to the experiment) have been added. If such a number of low molecular weight compounds have been added, the process proceeds to step 113, and if such a number of low molecular weight compounds have not been added, the process returns to step 108 to correct the estimated physical property value (specifically, increase or decrease the estimate value) calculated in S110 of the low molecular weight compounds determined in step 111.

In step 113 (S113), the experimenter carries out the experiment by using the low molecular weight compounds determined in S107 and the low molecular weight compounds determined in S111. When the objective is achieved as a result of the experiment (when the measured value of the physical property value satisfies the target value of the physical property value), the process is ended, and when the objective is not achieved, the process returns to step 109, and the regression model is generated again by adding the values obtained by the experiment in S113 (that is, the compound search apparatus 10 adds the chemical structure of the low molecular weight compound subjected to the experiment at S113 and the measured physical property value of the low molecular weight compound subjected to the experiment at S113 (the value obtained by the experiment at S113) to the data set for machine learning and relearns the regression model).

### Reference Signs List

1 compound search system
10 compound search apparatus
20 user terminal
30 user
101 control unit
102 ROM
103 RAM
104 auxiliary storage device
105 display device
106 operation device
107 I/F device
108 drive device
109 bus
110 storage medium

## Claims

1. A method executed by a computer, the method comprising:
a step of acquiring a chemical structure of a plurality of low molecular weight compounds (S1), wherein a low molecular weight compound is a compound having a molecular weight of 10,000 or less;
a step of replacing the chemical structure of a plurality of low molecular weight compounds with a plurality of types of descriptors (S2);
a step of selecting a most diverse group of compounds from among the plurality of the low molecular weight compounds, by selecting a combination in which det (X^{T}X) is maximum for the plurality of types of descriptors (S3) , wherein (X^{T}X) is the D optimization criterion,
a step of selecting, with respect to the plurality of types of the descriptors, at least one of the descriptors from among highly correlated descriptors, wherein
the most diverse group of compounds is selected by using the selected at least one of the descriptors, and
a number of the selected at least one of the descriptors is less than or equal to a number of the low molecular weight compounds forming the most diverse group of compounds,
wherein the most diverse group of compounds is selected by using a genetic algorithm,
a step of generating a regression model, wherein the regression model is a Gaussian process regression model, by using the chemical structure of the low molecular weight compounds forming the most diverse group of compounds and a measured value of a physical property value of the low molecular weight compounds forming the most diverse group (S5),
a step of calculating an estimate value of a physical property value of each of the low molecular weight compounds and a variation of the estimate values, from the acquired chemical structure of each of the low molecular weight compounds, by using the regression model (S6), wherein the regression model is a Gaussian process regression model; and
a step of calculating an acquisition function from the estimate values of the physical property value and the variation of the estimate values (S7),
a step of determining the low molecular weight compound for which an expected improvement is maximum, in a case where the acquisition function is the expected improvement.

2. The method according to claim 1, further comprising:
a step of selecting at least one of the low molecular weight compounds from among the plurality of the low molecular weight compounds (S3); and
a step of generating the regression model by using the chemical structure of the selected at least one of the low molecular weight compounds and a measured value of the physical property value of the selected at least one of the low molecular weight compounds (S5).

3. The method according to claim 1, further comprising:
a step of replacing the chemical structures of the plurality of the low molecular weight compounds with a plurality of types of descriptors (S2);
a step of selecting at least one of the low molecular weight compounds from among the plurality of the low molecular weight compounds based on the plurality of types of descriptors (S3); and
a step of generating the regression model by using the chemical structure of the selected at least one of the low molecular weight compounds and a measured value of the physical property value of the selected at least one of the low molecular weight compounds (S5).

4. The method according to claim 3, further comprising:
a step of converting the chemical structures of the plurality of the low molecular weight compounds into character strings.

5. The method according to claim 3, further comprising:
a step of replacing the chemical structures of the plurality of the low molecular weight compounds with a plurality of types of RDKit2D descriptors.

6. The method according to any one of claims 1 to 5, wherein the estimate value is an average value of a probability distribution, and the variation is a standard deviation of the probability distribution.

7. The method according to any one of claims 1 to 6, wherein the plurality of the low molecular weight compounds include at least one of a compound selected by a human, a compound extracted from a database, or a compound designed by a predetermined algorithm.

8. The method according to any one of claims 1 to 7, further comprising:
a step of correcting the estimate value of the physical property value (S108); and
a step of generating the regression model by using the chemical structure of the low molecular weight compound for which the estimate value of the physical property value has been corrected, and the corrected estimate value of the physical property value (S110).

9. A program that causes a computer to execute:
a procedure of acquiring a chemical structure of a plurality of low molecular weight compounds (S1) , wherein a low molecular weight compound is a compound having a molecular weight of 10,000 or less ;
a procedure of replacing the chemical structure of a plurality of low molecular weight compounds with a plurality of types of descriptors (S2);
a procedure of selecting a most diverse group of compounds from among the plurality of the low molecular weight compounds, by selecting a combination in which det (X^{T}X) is maximum for the plurality of types of descriptors (S3) , wherein (X^{T}X) is the D optimization criterion,
a procedure of selecting, with respect to the plurality of types of the descriptors, at least one of the descriptors from among highly correlated descriptors, wherein
the most diverse group of compounds is selected by using the selected at least one of the descriptors, and
a number of the selected at least one of the descriptors is less than or equal to a number of the low molecular weight compounds forming the most diverse group of compounds,
wherein the most diverse group of compounds is selected by using a genetic algorithm,
a procedure of generating a regression model, wherein the regression model is a Gaussian process regression model, by using the chemical structure of the low molecular weight compounds forming the most diverse group of compounds and a measured value of a physical property value of the low molecular weight compounds forming the most diverse group (S5),
a procedure of calculating an estimate value of a physical property value of each of the low molecular weight compounds and a variation of the estimate values, from the acquired chemical structure of each of the low molecular weight compounds, by using the regression model (S6), wherein the regression model is a Gaussian process regression model ; and
a procedure of calculating an acquisition function from the estimate values of the physical property value and the variation of the estimate values (S7),
a procedure of determining the low molecular weight compound for which an expected improvement is maximum, in a case where the acquisition function is the expected improvement.

10. An apparatus (10) comprising a control unit (101), wherein the control unit (101) is configured to
acquire a chemical structure of a plurality of low molecular weight compounds, wherein a low molecular weight compound is a compound having a molecular weight of 10,000 or less;
replace the chemical structure of a plurality of low molecular weight compounds with a plurality of types of descriptors (S2);
select a most diverse group of compounds from among the plurality of the low molecular weight compounds, by selecting a combination in which det (X^{T}X) is maximum for the plurality of types of descriptors (S3) , wherein (X^{T}X) is the D optimization criterion,
select, with respect to the plurality of types of the descriptors, at least one of the descriptors from among highly correlated descriptors, wherein
the most diverse group of compounds is selected by using the selected at least one of the descriptors, and
a number of the selected at least one of the descriptors is less than or equal to a number of the low molecular weight compounds forming the most diverse group of compounds,
wherein the most diverse group of compounds is selected by using a genetic algorithm,
generate a regression model, wherein the regression model is a Gaussian process regression model, by using the chemical structure of the low molecular weight compounds forming the most diverse group of compounds and a measured value of a physical property value of the low molecular weight compounds forming the most diverse group (S5),
calculate an estimate value of a physical property value of each of the low molecular weight compounds and a variation of the estimate values, from the acquired chemical structure of each of the low molecular weight compounds, by using the regression model, wherein the regression model is a Gaussian process regression model; and
calculate an acquisition function from the estimate values of the physical property value and the variation of the estimate values,
determine the low molecular weight compound for which an expected improvement is maximum, in a case where the acquisition function is the expected improvement.

11. A system comprising a server and a user terminal (20), wherein
the server includes a control unit (101) configured to
acquire a chemical structure of a plurality of low molecular weight compounds, wherein a low molecular weight compound is a compound having a molecular weight of 10,000 or less;
replace the chemical structure of a plurality of low molecular weight compounds with a plurality of types of descriptors (S2);
select a most diverse group of compounds from among the plurality of the low molecular weight compounds, by selecting a combination in which det (X^{T}X) is maximum for the plurality of types of descriptors (S3) , wherein (X^{T}X) is the D optimization criterion,
select, with respect to the plurality of types of the descriptors, at least one of the descriptors from among highly correlated descriptors, wherein
the most diverse group of compounds is selected by using the selected at least one of the descriptors, and
a number of the selected at least one of the descriptors is less than or equal to a number of the low molecular weight compounds forming the most diverse group of compounds,
wherein the most diverse group of compounds is selected by using a genetic algorithm,
generate a regression model, wherein the regression model is a Gaussian process regression model, by using the chemical structure of the low molecular weight compounds forming the most diverse group of compounds and a measured value of a physical property value of the low molecular weight compounds forming the most diverse group (S5),
calculate an estimate value of a physical property value of each of the low molecular weight compounds and a variation of the estimate values, from the acquired chemical structure of each of the low molecular weight compounds, by using the regression model, wherein the regression model is a Gaussian process regression model;
calculate an acquisition function from the estimate values of the physical property value and the variation of the estimate values; and
present the acquisition function to the user terminal (20),
determine the low molecular weight compound for which an expected improvement is maximum, in a case where the acquisition function is the expected improvement.

## Patentansprüche

1. Verfahren, das von einem Computer ausgeführt wird, wobei das Verfahren umfasst:
einen Schritt zum Erfassen einer chemischen Struktur einer Vielzahl von Verbindungen mit niedrigem Molekulargewicht (S1), wobei eine Verbindung mit niedrigem Molekulargewicht eine Verbindung ist, die ein Molekulargewicht von 10.000 oder weniger aufweist;
einen Schritt zum Ersetzen der chemischen Struktur einer Vielzahl von Verbindungen mit niedrigem Molekulargewicht durch eine Vielzahl von Deskriptorentypen (S2);
einen Schritt zum Auswählen einer vielfältigsten Gruppe von Verbindungen aus der Vielzahl der Verbindungen mit niedrigem Molekulargewicht, durch Auswählen einer Kombination, bei der det (*X^{T}X*) ein Maximum für die Vielzahl von Deskriptortypen (S3) ist, wobei (*X^{T}X*) das D-Optimierungskriterium ist,
einen Schritt zum Auswählen, in Bezug auf die Vielzahl von Deskriptortypen, mindestens eines der Deskriptoren aus hoch korrelierten Deskriptoren, wobei
die vielfältigste Gruppe von Verbindungen durch Verwenden des ausgewählten mindestens eines der Deskriptoren ausgewählt wird, und
eine Anzahl der ausgewählten mindestens einen der Deskriptoren kleiner als oder gleich einer Anzahl der Verbindungen mit niedrigem Molekulargewicht ist, welche die vielfältigste Gruppe von Verbindungen bilden,
wobei die vielfältigste Gruppe von Verbindungen durch Verwendung eines genetischen Algorithmus ausgewählt wird,
einen Schritt zum Erzeugen eines Regressionsmodells, wobei das Regressionsmodell ein Gauß'sches Prozessregressionsmodell ist, durch Verwenden der chemischen Struktur der Verbindungen mit niedrigem Molekulargewicht, welche die vielfältigste Gruppe von Verbindungen und einen gemessenen Wert eines physikalischen Eigenschaftswerts der Verbindungen mit niedrigem Molekulargewicht bilden, welche die vielfältigste Gruppe (S5) bilden,
einen Schritt zum Berechnen eines Schätzwerts eines physikalischen Eigenschaftswerts jeder der Verbindungen mit niedrigem Molekulargewicht und einer Variation der Schätzwerte aus der erfassten chemischen Struktur jeder der Verbindungen mit niedrigem Molekulargewicht unter Verwendung des Regressionsmodells (S6); und
einen Schritt zum Berechnen einer Akquisitionsfunktion aus den Schätzwerten des physikalischen Eigenschaftswerts und der Variation der Schätzwerte (S7),
einen Schritt zum Bestimmen der Verbindung mit niedrigem Molekulargewicht, für die eine erwartete Verbesserung maximal ist, in einem Fall, in dem die Akquisitionsfunktion die erwartete Verbesserung ist.

2. Verfahren nach Anspruch 1, weiter umfassend:
einen Schritt zum Auswählen mindestens einer der Verbindungen mit niedrigem Molekulargewicht aus der Vielzahl der Verbindungen mit niedrigem Molekulargewicht (S3); und
einen Schritt zum Erzeugen des Regressionsmodells unter Verwendung der chemischen Struktur der ausgewählten mindestens einen der Verbindungen mit niedrigem Molekulargewicht und eines gemessenen Wertes des physikalischen Eigenschaftswerts der ausgewählten mindestens einen der Verbindungen mit niedrigem Molekulargewicht (S5).

3. Verfahren nach Anspruch 1, weiter umfassend:
einen Schritt zum Ersetzen der chemischen Strukturen der Vielzahl der Verbindungen mit niedrigem Molekulargewicht durch eine Vielzahl von Deskriptortypen (S2);
einen Schritt zum Auswählen von mindestens einer der Verbindungen mit niedrigem Molekulargewicht aus der Vielzahl der Verbindungen mit niedrigem Molekulargewicht auf der Grundlage der Vielzahl von Deskriptortypen (S3); und
einen Schritt zum Erzeugen des Regressionsmodells durch Verwenden der chemischen Struktur der ausgewählten mindestens einen der Verbindungen mit niedrigem Molekulargewicht und eines gemessenen Wertes des physikalischen Eigenschaftswerts der ausgewählten mindestens einen der Verbindungen mit niedrigem Molekulargewicht (S5).

4. Verfahren nach Anspruch 3, weiter umfassend:
einen Schritt zum Umwandeln der chemischen Strukturen der Vielzahl der Verbindungen mit niedrigem Molekulargewicht in Zeichenketten.

5. Verfahren nach Anspruch 3, weiter umfassend:
einen Schritt zum Ersetzen der chemischen Strukturen der Vielzahl der Verbindungen mit niedrigem Molekulargewicht durch eine Vielzahl von RDKit2D-Deskriptortypen.

6. Verfahren nach einem der Ansprüche 1 bis 5, wobei der Schätzwert ein Durchschnittswert einer Wahrscheinlichkeitsverteilung ist, und die Variation eine Standardabweichung der Wahrscheinlichkeitsverteilung ist.

7. Verfahren nach einem der Ansprüche 1 bis 6, wobei die Vielzahl der Verbindungen mit niedrigem Molekulargewicht mindestens eine von einem Menschen ausgewählte Verbindung, eine aus einer Datenbank extrahierte Verbindung oder eine durch einen vorbestimmten Algorithmus entworfene Verbindung einschließt.

8. Verfahren nach einem der Ansprüche 1 bis 7, weiter umfassend:
einen Schritt zum Korrigieren des Schätzwerts des physikalischen Eigenschaftswerts (S108); und
einen Schritt zum Erzeugen des Regressionsmodells durch Verwenden der chemischen Struktur der Verbindung mit niedrigem Molekulargewicht, für die der Schätzwert des physikalischen Eigenschaftswerts korrigiert worden ist, und des korrigierten Schätzwerts des physikalischen Eigenschaftswerts (S110).

9. Programm, das einen Computer veranlasst, Folgendes auszuführen:
eine Prozedur zum Erfassen einer chemischen Struktur einer Vielzahl von Verbindungen mit niedrigem Molekulargewicht (S1), wobei eine Verbindung mit niedrigem Molekulargewicht eine Verbindung ist, die ein Molekulargewicht von 10.000 oder weniger aufweist;
eine Prozedur zum Ersetzen der chemischen Struktur einer Vielzahl von Verbindungen mit niedrigem Molekulargewicht durch eine Vielzahl von Deskriptorentypen (S2);
eine Prozedur zum Auswählen einer vielfältigsten Gruppe von Verbindungen aus der Vielzahl der Verbindungen mit niedrigem Molekulargewicht, durch Auswählen einer Kombination, bei der det (*X^{T}X*) ein Maximum für die Vielzahl von Deskriptortypen (S3) ist, wobei (*X^{T}X*) das D-Optimierungskriterium ist,
eine Prozedur zum Auswählen, in Bezug auf die Vielzahl von Deskriptortypen, mindestens einer der Deskriptoren aus hoch korrelierten Deskriptoren, wobei
die vielfältigste Gruppe von Verbindungen durch Verwenden des ausgewählten mindestens einen der Deskriptoren ausgewählt wird, und
eine Anzahl der ausgewählten mindestens einen der Deskriptoren kleiner als oder gleich einer Anzahl der Verbindungen mit niedrigem Molekulargewicht ist, welche die vielfältigste Gruppe von Verbindungen bilden,
wobei die vielfältigste Gruppe von Verbindungen durch Verwendung eines genetischen Algorithmus ausgewählt wird,
eine Prozedur zum Erzeugen eines Regressionsmodells, wobei das Regressionsmodell ein Gauß'sches Prozessregressionsmodell ist, durch Verwenden der chemischen Struktur der Verbindungen mit niedrigem Molekulargewicht, welche die vielfältigste Gruppe von Verbindungen und einen gemessenen Wert eines physikalischen Eigenschaftswerts der Verbindungen mit niedrigem Molekulargewicht bilden, welche die vielfältigste Gruppe (S5) bilden,
eine Prozedur zum Berechnen eines Schätzwerts eines physikalischen Eigenschaftswerts jeder der Verbindungen mit niedrigem Molekulargewicht und einer Variation der Schätzwerte aus der erfassten chemischen Struktur jeder der Verbindungen mit niedrigem Molekulargewicht durch Verwenden des Regressionsmodells (S6), wobei das Regressionsmodell ein Gauß'sches Prozessregressionsmodell ist; und
eine Prozedur zum Berechnen einer Akquisitionsfunktion aus den Schätzwerten des physikalischen Eigenschaftswerts und der Variation der Schätzwerte (S7),
eine Prozedur zum Bestimmen der Verbindung mit niedrigem Molekulargewicht, für die eine erwartete Verbesserung maximal ist, in einem Fall, in dem die Akquisitionsfunktion die erwartete Verbesserung ist.

10. Einrichtung (10), die eine Steuereinheit (101) umfasst, wobei die Steuereinheit (101) ausgebildet ist, um
eine chemische Struktur einer Vielzahl von Verbindungen mit niedrigem Molekulargewicht zu erfassen, wobei eine Verbindung mit niedrigem Molekulargewicht eine Verbindung ist, die ein Molekulargewicht von 10.000 oder weniger aufweist;
die chemische Struktur einer Vielzahl von Verbindungen mit niedrigem Molekulargewicht durch eine Vielzahl von Deskriptortypen (S2) zu ersetzen;
eine vielfältigste Gruppe von Verbindungen aus der Vielzahl der Verbindungen mit niedrigem Molekulargewicht auszuwählen, durch Auswählen einer Kombination, bei der det (X^{T}X) ein Maximum für die Vielzahl von Deskriptortypen (S3) ist, wobei (*X^{T}X*) das D-Optimierungskriterium ist,
in Bezug auf die Vielzahl von Deskriptortypen, mindestens eines der Deskriptoren aus hoch korrelierten Deskriptoren auszuwählen, wobei
die vielfältigste Gruppe von Verbindungen durch Verwenden des ausgewählten mindestens einen der Deskriptoren ausgewählt wird, und
eine Anzahl der ausgewählten mindestens einen der Deskriptoren kleiner als oder gleich einer Anzahl der Verbindungen mit niedrigem Molekulargewicht ist, welche die vielfältigste Gruppe von Verbindungen bilden,
wobei die vielfältigste Gruppe von Verbindungen durch Verwendung eines genetischen Algorithmus ausgewählt wird,
ein Regressionsmodell zu erzeugen, wobei das Regressionsmodell ein Gauß'sches Prozessregressionsmodell ist, durch Verwenden der chemischen Struktur der Verbindungen mit niedrigem Molekulargewicht, welche die vielfältigste Gruppe von Verbindungen und einen gemessenen Wert eines physikalischen Eigenschaftswerts der Verbindungen mit niedrigem Molekulargewicht bilden, welche die vielfältigste Gruppe (S5) bilden,
einen Schätzwert eines physikalischen Eigenschaftswerts jeder der Verbindungen mit niedrigem Molekulargewicht und eine Variation der Schätzwerte aus der erfassten chemischen Struktur jeder der Verbindungen mit niedrigem Molekulargewicht durch Verwenden des Regressionsmodells zu berechnen, wobei das Regressionsmodell ein Gauß'sches Prozessregressionsmodell ist; und
eine Akquisitionsfunktion aus den Schätzwerten des physikalischen Eigenschaftswerts und der Variation der Schätzwerte zu berechnen,
die Verbindung mit niedrigem Molekulargewicht, für die eine erwartete Verbesserung maximal ist, in einem Fall zu bestimmen, in dem die Akquisitionsfunktion die erwartete Verbesserung ist.

11. System, umfassend einen Server und ein Benutzerendgerät (20), wobei
der Server eine Steuereinheit (101) einschließt, die ausgebildet ist, um
eine chemische Struktur einer Vielzahl von Verbindungen mit niedrigem Molekulargewicht zu erfassen, wobei eine Verbindung mit niedrigem Molekulargewicht eine Verbindung ist, die ein Molekulargewicht von 10.000 oder weniger aufweist;
die chemische Struktur einer Vielzahl von Verbindungen mit niedrigem Molekulargewicht durch eine Vielzahl von Deskriptortypen (S2) zu ersetzen;
eine vielfältigste Gruppe von Verbindungen aus der Vielzahl der Verbindungen mit niedrigem Molekulargewicht auszuwählen, durch Auswählen einer Kombination, bei der det (*X^{T}X*) ein Maximum für die Vielzahl von Deskriptortypen (S3) ist, wobei (*X^{T}X*) das D-Optimierungskriterium ist,
in Bezug auf die Vielzahl von Deskriptortypen, mindestens einen der Deskriptoren aus hoch korrelierten Deskriptoren auszuwählen, wobei
die vielfältigste Gruppe von Verbindungen durch Verwenden des ausgewählten mindestens einen der Deskriptoren ausgewählt wird, und
eine Anzahl der ausgewählten mindestens einen der Deskriptoren kleiner als oder gleich einer Anzahl der Verbindungen mit niedrigem Molekulargewicht, welche die vielfältigste Gruppe von Verbindungen bilden, ist,
wobei die vielfältigste Gruppe von Verbindungen durch Verwendung eines genetischen Algorithmus ausgewählt wird,
ein Regressionsmodell zu erzeugen, wobei das Regressionsmodell ein Gauß'sches Prozessregressionsmodell ist, durch Verwenden der chemischen Struktur der Verbindungen mit niedrigem Molekulargewicht, welche die vielfältigste Gruppe von Verbindungen und einen gemessenen Wert eines physikalischen Eigenschaftswerts der Verbindungen mit niedrigem Molekulargewicht bilden, welche die vielfältigste Gruppe (S5) bilden,
einen Schätzwert eines physikalischen Eigenschaftswerts jeder der Verbindungen mit niedrigem Molekulargewicht und eine Variation der Schätzwerte aus der erfassten chemischen Struktur jeder der Verbindungen mit niedrigem Molekulargewicht durch Verwenden des Regressionsmodells zu berechnen, wobei das Regressionsmodell ein Gauß'sches Prozessregressionsmodell ist;
eine Akquisitionsfunktion aus den Schätzwerten des physikalischen Eigenschaftswerts und der Variation der Schätzwerte zu berechnen; und
die Akquisitionsfunktion dem Benutzerendgerät (20) zu präsentieren,
die Verbindung mit niedrigem Molekulargewicht, für die eine erwartete Verbesserung maximal ist, in einem Fall zu bestimmen, in dem die Akquisitionsfunktion die erwartete Verbesserung ist.

## Revendications

1. Procédé mis en œuvre par un ordinateur, le procédé comprenant :
une étape d'acquisition d'une structure chimique d'une pluralité de composés de faible poids moléculaire (S1), dans lequel un composé de faible poids moléculaire est un composé présentant un poids moléculaire inférieur ou égal à 10 000 ;
une étape de remplacement de la structure chimique d'une pluralité de composés de faible poids moléculaire par une pluralité de types de descripteurs (S2) ;
une étape de sélection d'un groupe le plus diversifié de composés parmi la pluralité des composés de faible poids moléculaire, en sélectionnant une combinaison dans laquelle det (X^{T}X) est maximal pour la pluralité de types de descripteurs (S3), dans lequel (X^{T}X) est le critère d'optimisation D,
une étape de sélection, en ce qui concerne la pluralité de types des descripteurs, d'au moins un des descripteurs parmi des descripteurs fortement corrélés, dans lequel
le groupe de composés le plus diversifié est sélectionné en utilisant l'au moins un descripteur sélectionné parmi les descripteurs, et
un nombre de l'au moins un descripteur sélectionné parmi les descripteurs est inférieur ou égal à un nombre des composés de faible poids moléculaire formant le groupe le plus diversifié de composés,
dans lequel le groupe le plus diversifié de composés est sélectionné en utilisant un algorithme génétique,
une étape de génération d'un modèle de régression, dans lequel le modèle de régression est un modèle de régression par processus gaussien, en utilisant la structure chimique des composés de faible poids moléculaire formant le groupe le plus diversifié de composés et une valeur mesurée d'une valeur de propriété physique des composés de faible poids moléculaire formant le groupe le plus diversifié (S5),
une étape de calcul d'une valeur estimée d'une valeur de propriété physique de chacun des composés de faible poids moléculaire et d'une variation des valeurs estimées, à partir de la structure chimique acquise de chacun des composés de faible poids moléculaire, en utilisant le modèle de régression (S6) ; dans lequel le modèle de régression est un modèle de régression par processus gaussien ; et
une étape de calcul d'une fonction d'acquisition à partir des valeurs estimées de la valeur de propriété physique et de la variation des valeurs estimées (S7),
une étape de détermination du composé de faible poids moléculaire pour lequel une amélioration attendue est maximale, dans un cas où la fonction d'acquisition est l'amélioration attendue.

2. Procédé selon la revendication 1, comprenant en outre :
une étape de sélection d'au moins un des composés de faible poids moléculaire parmi la pluralité des composés de faible poids moléculaire (S3) ; et
une étape de génération du modèle de régression en utilisant la structure chimique de l'au moins un composé sélectionné des composés de faible poids moléculaire et une valeur mesurée de la valeur de propriété physique de l'au moins un composé sélectionné des composés de faible poids moléculaire (S5).

3. Procédé selon la revendication 1, comprenant en outre :
une étape de remplacement des structures chimiques de la pluralité des composés de faible poids moléculaire par une pluralité de types de descripteurs (S2);
une étape de sélection d'au moins un des composés de faible poids moléculaire parmi la pluralité des composés de poids moléculaire inférieur basée sur la pluralité de types de descripteurs (S3) ; et
une étape de génération du modèle de régression en utilisant la structure chimique de l'au moins un composé sélectionné des composés de faible poids moléculaire et une valeur mesurée de la valeur de propriété physique de l'au moins un composé sélectionné des composés de faible poids moléculaire (S5).

4. Procédé selon la revendication 3, comprenant en outre :
une étape de conversion des structures chimiques de la pluralité des composés de faible poids moléculaire en chaînes de caractères.

5. Procédé selon la revendication 3, comprenant en outre :
une étape de remplacement des structures chimiques de la pluralité des composés de faible poids moléculaire par une pluralité de types de descripteurs RDKit2D.

6. Procédé selon l'une quelconque des revendications 1 à 5, dans lequel la valeur estimée est une valeur moyenne d'une distribution de probabilité, et la variation est un écart type de la distribution de probabilité.

7. Procédé selon l'une quelconque des revendications 1 à 6, dans lequel la pluralité des composés de faible poids moléculaire inclut au moins un parmi un composé sélectionné par un humain, un composé extrait d'une base de données, ou un composé conçu par un algorithme prédéterminé.

8. Procédé selon l'une quelconque des revendications 1 à 7, comprenant en outre :
une étape de correction de la valeur estimée de la valeur de propriété physique (S108) ; et
une étape de génération du modèle de régression en utilisant la structure chimique du composé de faible poids moléculaire pour lequel la valeur estimée de la valeur de propriété physique a été corrigée, et la valeur estimée corrigée de la valeur de propriété physique (S110).

9. Programme qui amène un ordinateur à exécuter :
une procédure d'acquisition d'une structure chimique d'une pluralité de composés de faible poids moléculaire (S1), dans lequel un composé de faible poids moléculaire est un composé présentant un poids moléculaire inférieur ou égal à 10 000 ;
une procédure de remplacement de la structure chimique d'une pluralité de composés de faible poids moléculaire par une pluralité de types de descripteurs (S2) ;
une procédure de sélection d'un groupe le plus diversifié de composés parmi la pluralité des composés de faible poids moléculaire, en sélectionnant une combinaison dans laquelle det (X^{T}X) est maximal pour la pluralité de types de descripteurs (S3), dans lequel (X^{T}X) est le critère d'optimisation D,
une procédure de sélection, en ce qui concerne la pluralité de types des descripteurs, d'au moins un des descripteurs parmi des descripteurs fortement corrélés, dans lequel
le groupe de composés le plus diversifié est sélectionné en utilisant l'au moins un descripteur sélectionné parmi les descripteurs, et
un nombre de l'au moins un descripteur sélectionné parmi les descripteurs est inférieur ou égal à un nombre des composés de faible poids moléculaire formant le groupe le plus diversifié de composés,
dans lequel le groupe de composés le plus diversifié est sélectionné en utilisant un algorithme génétique,
une procédure de génération d'un modèle de régression, dans lequel le modèle de régression est un modèle de régression par processus gaussien, en utilisant la structure chimique des composés de faible poids moléculaire formant le groupe le plus diversifié de composés et une valeur mesurée d'une valeur de propriété physique des composés de faible poids moléculaire formant le groupe le plus diversifié (S5),
une procédure de calcul d'une valeur estimée d'une valeur de propriété physique de chacun des composés de faible poids moléculaire et d'une variation des valeurs estimées, à partir de la structure chimique acquise de chacun des composés de faible poids moléculaire, en utilisant le modèle de régression (S6), dans lequel le modèle de régression est un modèle de régression par processus gaussien ; et
une procédure de calcul d'une fonction d'acquisition à partir des valeurs estimées de la valeur de propriété physique et de la variation des valeurs estimées (S7),
une procédure de détermination du composé de faible poids moléculaire pour lequel une amélioration attendue est maximale, dans un cas où la fonction d'acquisition est l'amélioration attendue.

10. Appareil (10) comprenant une unité de commande (101), dans lequel l'unité de commande (101) est configurée pour
acquérir une structure chimique d'une pluralité de composés de faible poids moléculaire, dans lequel un composé de faible poids moléculaire est un composé présentant un poids moléculaire inférieur ou égal à 10 000 ;
remplacer la structure chimique d'une pluralité de composés de faible poids moléculaire par une pluralité de types de descripteurs (S2) ;
sélectionner un groupe de composés le plus diversifié parmi la pluralité des composés de faible poids moléculaire, en sélectionnant une combinaison dans laquelle det (X^{T}X) est maximal pour la pluralité de types de descripteurs (S3), dans lequel (X^{T}X) est le critère d'optimisation D,
sélectionner, en ce qui concerne la pluralité de types des descripteurs, au moins un des descripteurs parmi des descripteurs fortement corrélés, dans lequel
le groupe de composés le plus diversifié est sélectionné en utilisant l'au moins un descripteur sélectionné parmi les descripteurs, et
un nombre de l'au moins un descripteur sélectionné parmi les descripteurs est inférieur ou égal à un nombre des composés de faible poids moléculaire formant le groupe le plus diversifié de composés,
dans lequel le groupe le plus diversifié de composés est sélectionné en utilisant un algorithme génétique,
générer un modèle de régression, dans lequel le modèle de régression est un modèle de régression par processus gaussien, en utilisant la structure chimique des composés de faible poids moléculaire formant le groupe le plus diversifié de composés et une valeur mesurée d'une valeur de propriété physique des composés de faible poids moléculaire formant le groupe le plus diversifié (S5),
calculer une valeur estimée d'une valeur de propriété physique de chacun des composés de faible poids moléculaire et une variation des valeurs estimées, à partir de la structure chimique acquise de chacun des composés de faible poids moléculaire, en utilisant le modèle de régression, dans lequel le modèle de régression est un modèle de régression par processus gaussien ; et
calculer une fonction d'acquisition à partir des valeurs estimées de la valeur de propriété physique et de la variation des valeurs estimées,
déterminer le composé de faible poids moléculaire pour lequel une amélioration attendue est maximale, dans un cas où la fonction d'acquisition est l'amélioration attendue.

11. Système comprenant un serveur et un terminal utilisateur (20), dans lequel
le serveur inclut une unité de commande (101) configurée pour
acquérir une structure chimique d'une pluralité de composés de faible poids moléculaire, dans lequel un composé de faible poids moléculaire est un composé présentant un poids moléculaire inférieur ou égal à 10 000 ;
remplacer la structure chimique d'une pluralité de composés de faible poids moléculaire par une pluralité de types de descripteurs (S2) ;
sélectionner un groupe le plus diversifié de composés parmi la pluralité des composés de faible poids moléculaire, en sélectionnant une combinaison dans laquelle det (X^{T}X) est maximal pour la pluralité de types de descripteurs (S3), dans lequel (X^{T}X) est le critère d'optimisation D,
sélectionner, en ce qui concerne la pluralité de types des descripteurs, au moins un des descripteurs parmi des descripteurs fortement corrélés, dans lequel
le groupe de composés le plus diversifié est sélectionné en utilisant l'au moins un descripteur sélectionné parmi les descripteurs, et
un nombre de l'au moins un descripteur sélectionné parmi les descripteurs est inférieur ou égal à un nombre des composés de faible poids moléculaire formant le groupe le plus diversifié de composés,
dans lequel le groupe le plus diversifié de composés est sélectionné en utilisant un algorithme génétique,
générer un modèle de régression, dans lequel le modèle de régression est un modèle de régression par processus gaussien, en utilisant la structure chimique des composés de faible poids moléculaire formant le groupe le plus diversifié de composés et une valeur mesurée d'une valeur de propriété physique des composés de faible poids moléculaire formant le groupe le plus diversifié (S5),
calculer une valeur estimée d'une valeur de propriété physique de chacun des composés de faible poids moléculaire et une variation des valeurs estimées, à partir de la structure chimique acquise de chacun des composés de faible poids moléculaire, en utilisant le modèle de régression, dans lequel le modèle de régression est un modèle de régression par processus gaussien ;
calculer une fonction d'acquisition à partir des valeurs estimées de la valeur de propriété physique et de la variation des valeurs estimées ; et
présenter la fonction d'acquisition au terminal utilisateur (20),
déterminer le composé de faible poids moléculaire pour lequel une amélioration attendue est maximale, dans un cas où la fonction d'acquisition est l'amélioration attendue.
